# FASCICULE DE BREVET EUROPEEN

(11) **EP 1 322 411 B1**
(45) Date de publication et mention de la délivrance du brevet: **17.01.2007**
(21) Numéro de dépôt: 01974445.7
(22) Date de dépôt: 05.10.2001
(51) Int. Cl.: B01J 13/00, A61K 9/51

(54) **SUSPENSION COLLOIDALE DE PARTICULES SUBMICRONIQUES DE VECTORISATION DE PRINCIPES ACTIFS ET LEUR MODE DE PREPARATION**
KOLLOIDALE SUSPENSION SUBMIKRONISCHER TEILCHEN DER VEKTORISATION AKTIVER PRINZIPIEN UND DEREN HERSTELLUNG
COLLOIDAL SUSPENSION OF SUBMICRONIC PARTICLES FOR CARRYING ACTIVE PRINCIPLES AND THEIR MODE OF PREPARATION

(30) Priorité: 06.10.2000 FR 0012837
(43) Date de publication de la demande: 02.07.2003
(73) Titulaire: FLAMEL TECHNOLOGIES, 69693 Venissieux Cédex (FR)
(72) Inventeur: BRYSON, Nathan, F-69390 Millery (FR); SOULA, Gérard, F-69330 Meyzieu (FR)
(74) Mandataire: Honoré, Anne-Claire
(86) Numéro de dépôt international: PCT/FR2001/003083
(87) Numéro de publication internationale: WO 2002/028521

(56) Documents cités:
- FR-A- 2 746 035
- FR-A- 2 801 226

## Description

### DOMAINE TECHNIQUE

Le domaine de la présente invention est celui des Particules de Vectorisation **(PV),** utiles pour l'administration de principes actifs **(PA).** Ces derniers sont, de préférence, des médicaments ou des nutriments pour l'administration à un organisme animal ou humain par voie orale ou nasale, vaginale, oculaire, sous-cutanée, intraveineuse, intramusculaire, intradermique, intrapéritonéale, intracérébrale, parentérale, etc... Mais il peut s'agir aussi de produits cosmétiques ou de produits phytosanitaires, tels que des herbicides, des pesticides, des insecticides, des fongicides, etc. En terme de nature chimique, les **PA** plus particulièrement, mais non limitativement, concernés par l'invention sont, par exemple, des protéines, des glycoprotéines, des peptides, des polysaccharides, des lipopolysaccharides, des oligonucléotides, des polynucléides et des molécules organiques.

La présente invention concerne, plus précisément, des suspensions colloïdales de Particules de Vectorisation, avantageusement de type submicronique, à base de polyaminoacides (PAA).

La présente invention vise aussi bien des particules nues en tant que telles, que les systèmes de vecteurs de **PA,** constitués par les particules chargées par le (ou les) **PA** considéré(s).

La présente invention a également trait à des solides pulvérulents comprenant ces **PV.**

L'invention concerne, également, des procédés de préparation desdites suspensions colloïdales de particules, avec ou sans **PA.**

### ART ANTERIEUR

L'encapsulation ou l'adsorption de **PA** dans les **PV** a notamment, pour but de modifier leur durée d'action et/ou de les acheminer au lieu du traitement et/ou augmenter la biodisponibilité desdits **PA.** De nombreuses techniques d'encapsulation ont déjà été proposées. De telles techniques visent, d'une part, à permettre le transport du **PA** jusqu'à son site d'action thérapeutique, tout en le protégeant contre les agressions de l'organisme (hydrolyse, digestion enzymatique, etc.) et, d'autre part, à contrôler la libération du **PA** sur son site d'action, afin de maintenir la quantité disponible pour l'organisme au niveau désiré. Les **PA** concernés par ces avatars de transport et de séjour dans l'organisme sont, par exemple, des protéines mais peuvent être, également, des produits tout autres, des molécules organiques d'origine synthétique ou naturelle. La revue de M.J. HUMPHREY (Delivery system for peptide Drugs, éditée par S. DAVIS et L. ILLUM, Plenum Press, N.Y. 1986), fait état de la problématique concernant l'amélioration de la biodisponibilité des **PA** et l'intérêt des systèmes de vectorisation et de libération contrôlée.

Les **PV** selon l'invention sont du type de celles sur lesquelles le **PA** est adsorbé.

Parmi tous les matériaux envisageables pour former des **PV,** les polymères sont de plus en plus utilisés, du fait de leurs propriétés intrinsèques. S'agissant du cahier des charges que l'on souhaite obtenir pour les **PV,** il est particulièrement exigeant et comprend, notamment, les spécifications suivantes.
1 La première spécification recherchée pour les **PV** serait que le polymère, constituant les **PV** soit biocompatible, éliminable (par excrétion) et/ou biodégradable et, encore mieux, qu'il soit métabolisé en produits non toxiques pour l'organisme. En outre, il conviendrait que la biodégradation dans l'organisme soit d'une durée suffisamment courte.
2 Les **PV** auraient avantage à pouvoir former, sans l'aide de solvant organique et/ou de tensioactif, une suspension aqueuse stable.
3 Il serait également souhaitable que les **PV** aient une taille suffisamment faible pour pouvoir subir, en suspension dans un liquide, une filtration stérilisante par un filtre dont le diamètre des pores est inférieur ou égal à 0,2 µm.
4 Il est souhaitable que les **PV** et les systèmes **PV-PA** puissent être obtenus par un procédé non dénaturant pour le **PA.**
5 Les **PV** devraient, avantageusement, permettre de contrôler la vitesse de libération du **PA.**
6 Une autre spécification importante serait que les systèmes **PV-PA** puissent constituer d'excellents médicaments injectables. Cette aptitude améliorée de l'administration par injection -e.g. intraveineuse ou intramusculaire- « injectabilité » se caractérise par :
   (i) un volume injecté réduit (pour une dose thérapeutique donnée)
   (ii) une viscosité faible.
   Ces deux propriétés sont satisfaites lorsque la dose thérapeutique de **PA** est associée à une quantité minimale de **PV.** En d'autres termes, les **PV** doivent avoir un fort taux de chargement en **PA.**
7 Le coût propre aux **PV** dans une préparation injectable doit être réduit et là encore il convient que les **PV** aient un fort taux de chargement en **PA.** En définitive, la faible taille et un fort taux de chargement sont des spécifications majeures recherchées pour les **PV.**
8 Il est également avantageux que le polymère, constitutif des **PV,** n'induise pas de réponse immunitaire.

Les propositions techniques antérieures, décrites infra, ont tenté de satisfaire l'ensemble de ces spécifications. A titre d'illustration, on citera les propositions antérieures (a) à (h) :
(a) Le brevet US-A-5 286 495 concerne un procédé d'encapsulation par vaporisation de protéines en phase aqueuse, à l'aide de matériaux ayant des charges opposées, à savoir : l'alginate (chargé négativement) et la polylysine (chargée positivement). Ce procédé de fabrication permet de produire des particules de taille supérieure à 35 µm.
(b) Par ailleurs, les techniques d'émulsion sont couramment utilisées pour préparer des microparticules chargées de **PA.** Par exemple, les demandes de brevets WO 91/06286, WO 91/06287 et WO 89/08449 divulguent de telles techniques d'émulsion dans lesquelles on a recours à des solvants organiques pour solubiliser des polymères, par exemple de type polylactique. Mais il s'est avéré que les solvants peuvent être dénaturants, notamment pour les **PA** peptidiques ou polypeptidiques.
(c) On connaît, également, des **PV** biocompatibles appelées protéinoïdes, décrites dès 1970 par X. FOX et K. DOSE dans « Molecular Evolution and the origin of Life », Ed. Marcel DEKKER Inc (1977). Ainsi, la demande de brevet WO 88/01213 propose un système à base d'un mélange de polypeptides synthétiques, dont la solubilité dépend du pH. Pour obtenir les microparticules matricielles selon cette invention, ils solubilisent le mélange de polypeptides, puis avec un changement de pH, ils provoquent la précipitation de particules protéinoides. Lorsque la précipitation s'effectue en présence d'un **PA,** celui-ci est encapsulé dans la particule.
(d) On mentionnera également, pour mémoire, le brevet US 4 351 337 qui relève d'un domaine différent de celui de la vectorisation de **PA** propre à l'invention. Ce brevet divulgue des implants massiques fixés et localisés à des endroits bien précis de l'organisme. Ces implants sont des tubes ou des capsules creuses de taille microscopiques (160 µm et de longueur égale à 2 000 µm), constitués de copolymères de copoly(aminoacides) -e.g. poly(acide glutamique-leucine) ou poly(benzylglutamate-leucine)- obtenus par copolymérisation de monomères de N-carboxyanhydrides d'aminoacides (NCA). L'inclusion d'un **PA** s'opère par une technique d'évaporation de solvant d'un mélange de polymère et de PA. Le brevet US 4 450 150 appartient à la même famille que le brevet US 4 351 337 étudié ci-dessus et a essentiellement le même objet. Les **PAA** constitutifs sont des poly(acide glutamique-éthylglutamate).
(e) La demande EP 0 734 720 a pour objet des particules de polyaminoacides utiles pour la vectorisation de **PA.** Ces particules ont une taille comprise entre 10 et 500 nm, de préférence entre 30 et 400 nm.
   Les particules selon EP 0 734 720 se forment spontanément par mise en contact de PAA avec une solution aqueuse. Les PAA comprennent des monomères aminoacides neutres et hydrophobes AANO (Leu, Ile, Val, Ala, Pro, Phe) et des monomères ionisables et hydrophiles AAI (Glu, Asp). Ces PAA sont préparés par copolymérisation de NCA de précurseurs d'AAI (e.g. Glu-OMe) et de NCA d'AAO (e.g. Leu) en solution dans un mélange dioxane/toluène. Le copoly(Glu-OMe)(Leu) obtenu en solution est récupéré par précipitation dans l'eau, filtration et séchage. Ce copolymère est alors soumis à une hydrolyse acide en l'incorporant dans l'acide TriFluoroAcétique (TFA), dans lequel il se dissout. Un copolymère (Glu-O-Na)(Leu) est récupéré après neutralisation, dialyse, filtration et lyophilisation.
   Le coPAA est dispersé dans une solution aqueuse de NaCl et il se forme spontanément une suspension de nanoparticules. Dès lors que l'on met en présence ces **PV** en suspension aqueuse avec un **PA,** celui-ci s'associe spontanément par adsorption avec les **PV.** Ces dernières présentent un coeur hydrophobe formé par des aminoacides hydrophobes et une "chevelure" extérieure hydrophile à base de d'aminoacides hydrophiles.
   Il est à noter que les particules de vectorisation selon EP 0 734 720 comportent des aminoacides ionisables (Glu) porteurs d'une charge électrique stabilisatrice négative, qui permet de prévenir la floculation et l'agrégation des particules **PV.**
(f) Le FR-A-2 746 035 concerne des microparticules de gel composite, physico-chimiquement stables, intègres et susceptibles d'être utilisées comme vecteurs de principes actifs. Ces microparticules sont constituées d'huile (I), de phase aqueuse (II) et d'au moins un copolyaminoacide (III) linéaire, non réticulé, synthétique et comportant au moins deux types différents de comonomères aminoacides AAI hydrophiles et AAO hydrophobes. Les AAI peuvent être : Glu, Asp, Or, Arg, Lys, Asn, His et leurs associations. Les comonomères AAO peuvent être : Leu, Tyr, Phé, Val, Cys, Ile et leurs associations.Le FR-A-2 746 035 décrit notamment page 27 lignes 8 à 18, une suspension colloïdale aqueuse de polyaminoacides, par exemple polyleucine/coglutamate de sodium.

Mais le FR-A-2 746 035 ne divulgue pas l'utilisation des copolymères seuls comme un système pharmaceutique nanoparticulaire de vectorisation de PA.

Les AAI du FR-A-2 746 035 ne sont pas des aminoacides hydrophiles neutres choisis dans le groupe comprenant :
✔ les acides aminés neutres naturels suivants : sérine, thréonine, hydroxyproline, glutamine ;
✔ les acides aminés neutres, rares ou synthétiques suivants : méthionine-Soxyde, O-glycosidyl-sérine ;
✔ les dérivés des acides aminés neutres : N-hydroxyéthyl-glutamine, N-hydroxypropyl-asparagine.

Toutes ces propositions techniques antérieures sus décrites :
o soit satisfont incomplètement aux spécifications du cahier des charges indiqué supra, et, en particulier une aptitude à la stérilisation par filtration, une haute vitesse de dégradation, une adaptabilité aux contraintes de l'administration de médicaments par injection, un faible coût et un fort taux de chargement en **PA ;**
o soit pourraient être remplacées par de nouvelles solutions techniques susceptibles de procurer de nouveaux avantages (référence antérieure EP 0 734 720).

### BREF EXPOSE DE L'INVENTION

Dans cet état de fait, un objectif essentiel est de pouvoir fournir de nouvelles **PV** qui forment spontanément, et sans l'aide de tensioactifs ou de solvants organiques, des suspensions aqueuses stables de **PV.**

Un autre objectif essentiel de la présente invention est de fournir de nouvelles **PV** en suspension aqueuse colloïdale stable ou sous forme pulvérulente et à base de poly(aminoacides) (PAA), ces nouvelles **PV** se devant de satisfaire au mieux aux spécifications 1 à 8 du cahier des charges susvisé.

Un autre objectif essentiel de l'invention est de perfectionner les particules divulguées dans la demande PCT WO 96/29991.

Un autre objectif essentiel de l'invention est de fournir une suspension nouvelle de **PV** dont on maîtrise parfaitement les caractéristiques, notamment en termes du taux de chargement en **PA** et en termes de contrôle de cinétique de libération du **PA.**

Un autre objectif essentiel de l'invention est de fournir des suspensions médicamenteuses injectables. Les spécifications, requises pour de telles suspensions, sont un faible volume d'injection et une faible viscosité. Il importe que la masse de particules colloïdales par dose d'injection soit le plus faible possible et ce sans limiter la quantité du principe actif **PA** transporté par ces particules, afin de ne pas nuire à l'efficacité thérapeutique.

Un autre objectif essentiel de l'invention est de fournir une suspension colloïdale aqueuse ou un solide pulvérulent comprenant des particules de vectorisation de principes actifs satisfaisant aux spécifications visées ci-dessus et qui constitue une forme galénique appropriée et convenable pour une administration, par exemple orale, à l'homme ou l'animal.

Un autre objectif essentiel de l'invention est de fournir une suspension colloïdale comprenant des particules de vectorisation de principes actifs filtrable sur des filtres de 0,2 µm à des fins de stérilisation.

Un autre objectif essentiel de l'invention est de proposer un procédé de préparation de particules (sèches ou en suspension dans un liquide) de PAA utiles, notamment, comme vecteurs de principes actifs, ledit procédé se devant d'être, plus simple à mettre en oeuvre, non dénaturant pour les principes actifs et devant en outre toujours permettre une maîtrise fine de la granulométrie moyenne des particules obtenues.

Un autre objectif essentiel de l'invention est l'utilisation des susdites particules en suspension aqueuse ou sous forme solide pour la préparation :
- de médicaments (e.g. vaccins), en particulier pour administration notamment orale, nasale, vaginale, oculaire, sous-cutanée, intraveineuse, intramusculaire, intradermique, intrapéritonéale, intracérébrale ou parentérale, les principes actifs de ces médicaments pouvant être, notamment, des protéines, des glycoprotéines, des peptides, des polysaccharides, des lipopolysaccharides, des oligonucléotides et des polynucléotides ;
- et/ou de nutriments ;
- et/ou de produits cosmétiques ou phytosanitaires ;
- et/ou de molécules organiques médicamenteuses.

Un autre objectif essentiel de la présente invention est de fournir des suspensions de **PV** submicroniques à base de PAA et susceptibles de servir de vecteur d'un **PA,** en particulier médicamenteux pour l'administration dudit **PA** à un organisme humain ou animal, ou bien encore d'un **PA** nutritionnel, phytosanitaire ou cosmétique.

Un autre objectif de la présente invention est de fournir un médicament, du type système à libération prolongée de principes actifs, qui soit aisé et économique à produire et qui soit, en outre, biocompatible et apte à assurer un très haut niveau de biodisponibilité du **PA.**

Un autre objectif essentiel de l'invention est de fournir un système de vectorisation de vaccin, qui soit non-immunogène intrinsèquement et en combinaison avec un ou plusieurs antigènes.

Les objectifs relatifs aux produits (parmi d'autres) sont atteints par la présente invention qui concerne, tout d'abord, une suspension colloïdale stable de particules structurées submicroniques susceptibles d'être utilisées, notamment pour la vectorisation de principe(s) actif(s) **PA,** ces particules étant des arrangements supramoléculaires individualisés (discrets) :
o à base de polyaminoacides (PAA) amphiphiles, linéaires, à enchaînements peptidiques et comprenant au moins deux types différents d'aminoacides récurrents hydrophiles AAI et hydrophobes AAO, les aminoacides de chaque type étant identiques ou différents entre eux ;
o aptes à associer en suspension colloïdale à l'état non dissous, au moins un **PA** et à libérer celui-ci, notamment in vivo, de manière prolongée et/ou retardée ;
o et stables en phase aqueuse à pH compris entre 4 et 13 en l'absence de tensioactif(s) ;
caractérisée en ce que
✔ en ce que les aminoacides récurrents hydrophiles AAI sont des aminoacides neutres hydrophiles AANI à l'exclusion de l'Asparagine;
✔ en ce que les aminoacides récurrents hydrophobes AAO sont des aminoacides neutres hydrophobes AANI ;
✔ et en ce que les acides aminés récurrents de chaque type AANI et AANO étant identiques ou différents entre eux.

### EXPOSE DETAILLE DE L'INVENTION

L'un des fondements inventifs de ces nouvelles particules de vectorisation **PV,** en suspension aqueuse colloïdale stable ou à l'état de solide pulvérulent, tient à la sélection originale d'un groupe de polymères et d'un méthodologie originale permettant d'obtenir des particules de taille submicronique, qui forment une suspension colloïdale aqueuse stable en l'absence de tensioactifs ou de solvants.

Un autre fondement inventif de ces nouvelles particules de vectorisation **PV,** en suspension aqueuse colloïdale stable ou à l'état de solide pulvérulent, tient à la sélection originale de deux aminoacides neutres à titre de monomères récurrents hydrophiles AANI et hydrophobes AANO.

Or, contrairement à ce que l'homme du métier aurait pu craindre, le fait de ne pas avoir d'aminoacides hydrophiles ionisables AAII et donc pas de charges négatives, comme dans les particules selon le WO 96/29991, n'a pas nuit à la stabilité. En effet, contre toute attente, la suspension colloïdale selon la présente invention ne flocule pas. Les particules à base de poly(AANI/AANO) ne s'auto-agrègent pas.

En outre, il n'était absolument pas évident a priori que ces particules de poly(AANI/AANO) puissent s'associer spontanément avec des principes actifs **PA** et relarguer ces **PA** sur les sites d'action thérapeutique.

La structure des polymères PAA et la nature des acides aminés neutres, sont choisies de telle façon que :
- les chaînes de polymères se structurent spontanément sous forme de particules **(PV)** de petite taille ;
- les particules forment une suspension colloïdale stable dans l'eau et en milieu physiologique ;
- les **PV** s'associent avec des protéines ou autres **PA** en milieu aqueux, par un mécanisme spontané et non dénaturant pour la protéine ;
- les **PV** libèrent les **PA** en milieu physiologique et, plus précisément, in vivo ; la cinétique de libération est fonction de la nature du polymère PAA précurseur des **PV.**

Ainsi, en jouant sur la structure particulière du PAA, on peut contrôler les phénomènes d'association et de libération du **PA** sur le plan cinétique et quantitatif.

Il est du mérite de la demanderesse d'avoir choisi, à titre de matériau constitutif des **PV,** une composition particulière de polyaminoacides neutres qui sont amphiphiles et qui, donc, possèdent des propriétés des **PV** en **PAA,** à savoir :
- possibilité de former spontanément des suspensions colloïdales de **PV** compatibles avec le pH des milieux physiologiques rencontrés dans les applications thérapeutiques visées ;
- association spontanée des **PA** avec des **PV** en l'absence d'autre agent que l'eau qui leur sert de solvant et qui, dans le cas des protéines, n'est pas dénaturant ;
- possibilité de libérer le **PA** du complexe d'association **PA-PV,** dans des conditions physiologiques, avec des profils pharmacocinétique et pharmacodynamique, qui laissent présager des utilisations intéressantes dans le domaine thérapeutique (vectorisation **PA) ;**
- filtrabilité avec seuil de coupure inférieur ou égal à 0,2 µm à des fins de stérilisation ;
- biodégradabilité améliorée ;
- aptitude à l'injection optimisée.

Ces PAA peuvent être du type ordonné, séquentiel alterné (blocs) ou du type désordonné, séquentiel aléatoire (statistiques).

Ainsi, selon une première forme de réalisation des **PV** selon l'invention, les PAA constitutifs sont du type « bloc » et sont caractérisés par un rapport molaire AANO/(AANI+AANO) tel que :
- AANO/(AANI+AANO)≥ 6%,
- 10% ≤ AANO/(AANO + AANI) ≤70%,
- de préférence, 20% AANO/(AANI + AANO) ≤60%,
- et plus préférentiellement encore, 35% ≤ AANO/(AANI + AANO) ≤ 50%.

Avantageusement, la longueur absolue de chaque bloc d'AANO, exprimé en nombre d'AANO est telle que :
- AANO ≥ 5,
- de préférence, AANO ≥ 10,
- et, plus préférentiellement encore, AANO ≥ 20.

Selon une deuxième forme de réalisation des **PV** selon l'invention, les PAA constitutifs sont du type « statistique » c'est-à-dire préparés par copolymérisation simultanée de monomères de AANI et AANO, et le rapport molaire AANO/(AANO + AANI) est tel que :
- AANO/(AANO + AANI) ≥ 10 %,
- et, de préférence, AANO/(AANO + AANI) ≥ 20 %,
- et, plus préférentiellement encore, 30% ≤ AANO/(AANI + AANO) ≤ 70%.

Avantageusement, la masse molaire Mw de ces PAA statistiques est telle que :
- Mw ≥ 2 000 g/mol,
- de préférence, Mw ≥ 5 500 g/mol,
- et plus, préférentiellement encore, 5 500 g/mol ≤ Mw ≤ 200 000 g/mol.

Suivant une caractéristique préférée de l'invention, les PAA blocs ou statistiques constitutifs de particules ont des degrés de polymérisation DP compris entre 30 et 600, de préférence entre 50 et 200 et, plus préférentiellement encore, entre 60 et 150.

Avantageusement, les PAA constitutifs des particules **PV** sont des PAA « diblocs ».

De préférence, l'(les)AANI hydrophile(s) est(sont) choisi(s) dans le groupe comprenant :
✔ les acides aminés neutres naturels, de préférence ceux choisis dans le groupe comprenant: sérine, thréonine, hydroxyproline, glutamine;
✔ les acides aminés neutres, rares ou synthétiques suivants, de préférence ceux choisis dans le groupe comprenant : méthionine-S-oxyde, O-glycosidyl-sérine;
✔ les dérivés des acides aminés neutres, de préférence ceux choisis dans le groupe comprenant : N-hydroxyéthylglutamine, N-hydroxypropylasparagine, N-hydroxyéthyl-asparagine, N-hydroxypropylglutamine.

Avantageusement, l'(les)AANO hydrophobe(s) est(sont) choisi(s) dans le groupe comprenant :
◆ les acides aminés neutres naturels, de préférence ceux choisis dans le groupe comprenant : Leu, Ile, Val, Ala, Pro, Phe ;
◆ les acides aminés neutres, rares ou synthétiques, de préférence ceux choisis dans le groupe comprenant : norleucine, norvaline ;
◆ les dérivés des acides aminés polaires, de préférence ceux choisis dans le groupe comprenant : glutamate de méthyle, glutamate d'éthyle, aspartate de benzyle, N-acétyllysine.

Suivant une caractéristique avantageuse, les particules de vectorisation (PV) de la suspension ont une de taille moyenne comprise entre 0,01 et 0,5 µm, de préférence entre 0,01 et 0,2 µm.

La suspension a également pour caractéristique préférée d'être aqueuse et stable.

La présente invention vise, non seulement des suspensions de particules nues, telles que définies ci-dessus, mais également des particules comprenant au moins un principe actif **PA.** De préférence, la suspension selon l'invention est aqueuse et stable. Ces particules, chargées ou non en **PA,** sont, avantageusement, sous forme dispersée dans un liquide (suspension), de préférence aqueux, mais peuvent également être à l'état de solide pulvérulent, obtenu à partir de la suspension de PV telle que définie ci-dessus.

D'où il s'ensuit que l'invention concerne, outre une suspension colloïdale (de préférence aqueuse) de **PV,** un solide pulvérulent comprenant des **PV** et obtenu à partir de la suspension selon l'invention.

Un autre objet essentiel de l'invention se rapporte à la préparation :
✦ des particules sélectionnées telles que décrites ci-avant
✦ et d'autres particules sélectionnées qui sont structurées, submicroniques et susceptibles d'être utilisées, notamment pour la vectorisation de principe(s) actif(s) **PA,** ces particules étant des arrangements supramoléculaires individualisés (discrets) :
   o à base de polyaminoacides (PAA) amphiphiles, linéaires, à enchaînements peptidiques et comprenant au moins deux types différents d'aminoacides récurrents hydrophiles AAI et hydrophobes AAO, les aminoacides de chaque type étant identiques ou différents entre eux ;
   o aptes à associer en suspension colloïdale à l'état non dissous, au moins un **PA** et à libérer celui-ci, notamment in vivo, de manière prolongée et/ou retardée;
   o et stables en phase aqueuse à pH compris entre 4 et 13 en l'absence de tensioactif(s);
dans laquelle :
✔ les aminoacides récurrents hydrophiles AAI sont au moins en partie constitués par de l'Asparagine;
✔ et les aminoacides récurrents hydrophobes AAO sont des aminoacides neutres hydrophobes AANO identiques ou différents entre eux;
ces particules pouvant être aussi bien sous forme de suspension colloïdale que sous forme de solide pulvérulent obtenu à partir d'une suspension colloïdale stable de particules.

Le procédé de préparation considéré consiste, essentiellement, à synthétiser des PAA précurseur et à les transformer en particules structurées.

Plus précisément, il s'agit, tout d'abord, d'un procédé de préparation de particules structurées submicroniques susceptibles d'être utilisées, notamment pour la vectorisation de principe(s) actif(s), ces particules étant des arrangements supramoléculaires discrets :
o à base de polyaminoacides (PAA) amphiphiles, linéaires, à enchaînements peptidiques et comprenant au moins deux types différents d'aminoacides récurrents hydrophiles AAI et hydrophobes AAO, les aminoacides de chaque type étant identiques ou différents entre eux ;
o aptes à associer en suspension colloïdale à l'état non dissous, au moins un **PA** et à libérer celui-ci, notamment in vivo, de manière prolongée et/ou retardée ;
o et stables en phase aqueuse à pH compris entre 4 et 13 en l'absence de tensioactif(s).

Ce procédé est caractérisé en ce que :
1) on réalise une copolymérisation de monomères formés par des anhydrides de N-CarboxyAminoacides (NCA) d'au moins deux types différents :
   ◆ d'une part, des monomères NCA de départ comprenant des NCA-Glu-OR et/ou des NCA-Asp-OR, et/ou des NCA-AANI,
   ◆ et d'autre part, des NCA-AANO, en présence:
      - d'au moins un solvant polaire non aromatique, de préférence choisi dans le groupe comprenant: la N-MéthyIPyrrolidone (NMP), le DiMéthyIFormamide (DMF), le DiMéthylsulfoxyde (DMSO), le DiMéthylAcétamide (DMAc), la pyrrolidone ; la NMP étant plus particulièrement préférées;
      - et éventuellement d'au moins un co-solvant sélectionné parmi les solvants aprotiques (de préférence le dioxanne-1,4) et/ou les solvants protiques (de préférence la pyrrolidone) et/ou l'eau et/ou les alcools, le méthanol étant particulièrement préféré ;
2) dans le cas où les monomères NCA de départ sont des NCA-Glu-OR et/ou des NCA-Asp-OR (R= alkyle), on met en oeuvre une aminolyse qui consiste à mettre en présence le copolymère obtenu à l'étape 1 avec une phase aqueuse comprenant au moins une amine et qui permet la transformation de Glu-OR en Gln et de Asp-OR en Asn ;
3) éventuellement on dialyse le milieu réactionnel pour purifier la suspension aqueuse de particules structurées ;
4) éventuellement on concentre cette suspension de l'étape 3;
5) on élimine le milieu liquide pour recueillir le solide pulvérulent comprenant les particules.

La première étape du procédé s'inspire des techniques connues de polymérisation d'anhydrides de N-carboxy-(-aminoacides (NCA), décrites, par exemple, dans l'article «Biopolymers, 15, 1869 (1976)» et dans l'ouvrage de H.R. KRICHELDORF «α-Aminoacid-N-carboxy Anhydride and Related Heterocycles» Springer Verlag (1987).

La mise en oeuvre de solvants de copolymérisation aprotiques non aromatiques polaire, judicieusement choisis, en évitant toute précipitation et le fait d'avoir recours à une hydrolyse acide en présence d'eau et de solvant organique polaire non aromatique, permet d'obtenir des particules structurées, discrètes et submicroniques à forte capacité de chargement de **PA,** et qui forment une suspension colloïdale, stable en milieu aqueux. Ces particules ne sont nullement comparables à un précipité aggloméré macroscopique du genre de celui évoqué ci-avant à propos de la proposition antérieure (d).

Suivant une variante, à l'issue de l'étape 1, on précipite - de préférence dans l'eau - le copolymère poly(AANO)(AANI) obtenu et on recueille ce précipité. Cette variante correspond à un mode discontinu de préparation de particules, dans lequel on isole le copolymère poly(AANO)(AANI) sous forme de précipité formant un produit intermédiaire stable. Ce précipité peut être, par exemple, filtré, lavé et séché.

De manière plus préférée encore, les NCA-pAAI sont des NCA d'acide glutamique ou aspartique O-alkylé, par exemple des NCA-Glu-O-Me, NCA-Glu-O-Et ou NCA-Glu-O-Bz (Me = méthyle - Et = Ethyle - Bz = Benzyle).

De manière connue, la copolymérisation se déroule à une température comprise entre 20 et 120°C, à pression atmosphérique et en présence d'un initiateur aminé, e.g. : NH₃.

D'autres paramètres expérimentaux, comme la concentration en NCA et/ou polymère dans le solvant polaire non aromatique (de préférence le NMP), et/ou la concentration ou la nature du cosolvant protique, lors de la synthèse, seront ajustés selon les effets désirés et connus de l'homme de l'art.

L'hydrolyse acide (étape 2) est réalisée à l'aide d'eau et d'au moins un acide minéral, tel l'aide phosphorique ou chlorhydrique - ce dernier étant préféré - et/ou un acide organique, tel l'acide TriFluoroAcétique (TFA), l'acide acétique, l'acide dichloroacétique, ou les acides organosulfoniques.

Les proportions eau/acide -exprimées en parties en poids- dans une phase aqueuse acide d'hydrolyse sont, avantageusement :
- de 60/1 à 2/1,
- de préférence 40/1 à 2/1,
- et, plus préférentiellement encore, 20/1 à 2/1.

Les proportions phase aqueuse acide d'hydrolyse/NMP - exprimées en parties en poids - sont, avantageusement :
- de 5/100 à 200/100
- de préférence, 10/100 à 100/100
- et, plus préférentiellement encore, de 20/100 à 80/100.

D'autres paramètres, comme la concentration en polymère, la température du mélange réactionnel, le mode d'ajout de la phase aqueuse acide d'hydrolyse, l'emploi de pression réduite, la durée de la réaction, etc..., sont ajustés selon les effets désirés et bien connus de l'homme de l'art.

La neutralisation (étape 3) s'opère, en pratique, par exemple à l'aide de soude.

On élimine ensuite le sel formé à l'issue de la neutralisation, ainsi que le solvant, par tout traitement de séparation physique approprié, par exemple par diafiltration (dialyse) (étape 4), filtration, modification pH, chromatographie...

Cela conduit à une suspension aqueuse de particules structurées qui peut être concentrée, par exemple par distillation ou tout autre moyen physique convenable : ultrafiltration, centrifugation. Pour séparer, à l'étape 6, les particules de leur milieu liquide de suspension, on élimine, éventuellement, la phase aqueuse, par exemple par séchage (e.g. à l'étuve), par lyophilisation ou tout autre moyen physique convenable : ultrafiltration, centrifugation. On récupère, à l'issue de cette étape 6, un solide pulvérulent, de couleur blanche.

Selon une variante, l'étape de concentration peut être réalisée par un traitement chimique, tel qu'un abaissement du pH, qui transforme en acide la partie hydrophile des monomères glutamates, ce qui les rend insolubles dans l'eau. Ces intermédiaires PAA acides peuvent être filtrés, lavés et séchés. Lesdits intermédiaires acides peuvent être neutralisés avec une base chimique dans une étape ultérieure afin d'obtenir une suspension de particules.

Il est à noter que la mise en oeuvre des étapes 1, 2, 3, 4 et éventuellement 5 du procédé ci-dessus correspondant à une préparation d'une suspension colloïdale de particules submicroniques et à fort taux de chargement avec les **PA.**

Lors de cette préparation de suspension colloïdale, les PAA amphiphiles poly(AANO)(AANI) de l'étape 2 sont placés dans un milieu aqueux dans lequel au moins une partie des AANI est soluble et au moins une partie des AANO est insoluble. Les PAA existent sous forme de nanoparticules dans ce milieu aqueux.

Une alternative pour préparer la suspension de **PV** selon l'invention consiste à mettre en présence le solide pulvérulent, tel que décrit ci-dessus et en tant que produit et par son procédé d'obtention, avec un milieu aqueux, non solvant des AANO.

Pour effectuer l'association d'un ou plusieurs **PA** aux particules, il est possible de mettre en oeuvre plusieurs méthodes conformément à l'invention. Des exemples, non limitatifs de ces méthodes, sont énumérés ci-après.

Selon une première méthode, on effectue l'association de **PA** aux particules par mise en présence d'une phase liquide (aqueuse ou non) contenant le **PA** avec la suspension colloïdale de particules comprenant ou non de l'Asp à titre d'AANI.

Selon une deuxième méthode, on effectue l'association du **PA** aux particules par mise en présence d'un **PA** à l'état solide avec la suspension colloïdale de particules comprenant ou non de l'Asp à titre d'AANI. Le **PA** solide peut être, par exemple, sous forme de lyophilisat, de précipité, de poudre ou autre.

Selon une troisième méthode, on met en présence le solide pulvérulent (PAA comprenant ou non de l'Asp à titre d'AANI), tel que décrit supra en tant que produit et par ses caractéristiques d'obtention, avec une phase liquide (aqueuse ou non) contenant le **PA.**

Selon une quatrième méthode, on met en présence le solide pulvérulent, tel que décrit supra en tant que produit et par ses caractéristiques d'obtention(PAA comprenant ou non de l'Asp à titre d'AANI), avec le **PA** sous forme solide. On disperse ensuite ce mélange de solides, dans une phase liquide, de préférence une solution aqueuse.

Dans toutes ces méthodes, le **PA** utilisé peut être sous forme pure ou préformulée.

Compte tenu de la taille nanométrique des particules, la suspension peut être filtrée sur des filtres de stérilisation, ce qui permet d'obtenir, aisément et à moindre coût, des liquides médicamenteux injectables stériles. Le fait de pouvoir, grâce à l'invention, contrôler la taille des particules et atteindre des valeurs de Dh entre 25 et 100 nm, est un atout important.

La présente invention vise, également, de nouveaux produits intermédiaires du procédé décrit ci-dessus, caractérisés en ce qu'ils sont constitués par des copolymères PAA(comprenant ou non de l'Asp à titre d'AANI) précurseurs de particules.

### APPLICATION INDUSTRIELLE

Selon un autre de ses aspects, l'invention concerne une suspension et/ou un solide pulvérulent(PAA comprenant ou non de l'Asp à titre d'AANI), tels que définis ci-dessus et/ou tels qu'obtenus par le procédé présenté supra, cette suspension et ce solide comprenant au moins un principe actif, choisi, de préférence, parmi :
- les vaccins ;
- les protéines et/ou les peptides, parmi lesquels les plus préférentiellement retenus sont : les hémoglobines, les cytochromes, les albumines, les interférons, les antigènes, les anticorps, l'érythropoïétine, l'insuline, les hormones de croissance, les facteurs VIII et IX, les interleukines ou leurs mélanges, les facteurs stimulants de l'hématopoïèse ;
- les polysaccharides, l'héparine étant plus particulièrement sélectionnée ;
- les acides nucléiques et, préférablement, les oligonucléotides d'ARN et/ou d'ADN ;
- des molécules non petido-protéiques appartenant à diverses classes de chimiothérapie anticancéreuses et, en particulier, les anthracyclines et les taxoïdes ;
- et leurs mélanges.

L'invention vise, également, une suspension et/ou le solide pulvérulent(PAA comprenant ou non de l'Asp à titre d'AANI) chargé(s) en **PA** nutritionnel, phytosanitaire ou cosmétique.

Enfin, l'invention concerne une spécialité pharmaceutique, nutritionnelle, phytosanitaire ou cosmétique, caractérisée en ce qu'elle comporte une suspension et/ou du solide pulvérulent chargé(s) en **PA** et tels que définis ci-dessus.

Selon un autre de ses objets, l'invention vise, également, l'utilisation de ces **PV** (en suspension ou sous forme solide:PAA comprenant ou non de l'Asp à titre d'AANI) chargées en **PA,** pour la fabrication de médicaments du type systèmes à libération contrôlée de **PA.**

En particulier, l'invention se rapporte à l'utilisation d'une suspension colloïdale stable de particules structurées submicroniques chargées en principe(s) actif(s) **PA,** ces particules étant des arrangements supramoléculaires individualisés (discrets) :
o à base de polyaminoacides (PAA) amphiphiles, linéaires, à enchaînements peptidiques et comprenant au moins deux types différents d'aminoacides récurrents hydrophiles AAI et hydrophobes AAO, les aminoacides de chaque type étant identiques ou différents entre eux ;
o aptes à associer en suspension colloïdale à l'état non dissous, au moins un **PA** et à libérer celui-ci, notamment in vivo, de manière prolongée et/ou retardée;
o et stables en phase aqueuse à pH compris entre 4 et 13 en l'absence de tensioactif(s);
dans laquelle:
✔ les aminoacides récurrents hydrophiles AAI sont au moins en partie constitués par de l'Asparagine;
✔ et les aminoacides récurrents hydrophobes AAO sont des aminoacides neutres hydrophobes AANO identiques ou différents entre eux;
pour la préparation d'une suspension aqueuse ou d'un solide pulvérulent, chargé en au moins PA et tel que défini dans les revendications précédentes.

Dans le cas de médicaments, il peut s'agir, par exemple de ceux administrables, de préférence par voie orale, nasale, vaginale, oculaire, sous-cutanée, intraveineuse, intramusculaire, intradermique, intrapéritonéale, intracérébrale ou parentérale.

Les applications cosmétiques envisageables sont, par exemple, les compositions comprenant un **PA** associé aux **PV** selon l'invention et applicables par voie transdermique.

Les produits phytosanitaires concernés peuvent être, par exemple, des herbicides, des pesticides, des insecticides, des fongicides, etc...

Les exemples qui suivent permettront de mieux comprendre l'invention dans ses différents aspects produit/procédé/application. Ces exemples illustrent la préparation de particules de polyaminoacides chargés ou non en principes actifs, de même qu'ils présentent les caractéristiques de structure et les propriétés de ces particules.

### EXEMPLES

### Exemple 1 - Préparation du polymère - poly(leucine)-bloc-(glutamate de méthyle)

Les techniques utilisées, pour la polymérisation des NCA en polymères de structures en blocs ou statistiques sont connues de l'homme de l'art et sont détaillées dans l'ouvrage de H. R. KRICHELDORF "α-Aminoacides-N-Carboxy Anhydrides and Related Heterocycles", Springer Verlag (1987). La synthèse suivante précise la synthèse de l'un d'entre eux.

*Synthèse de la poly(Leu)₄₀-poly(GluOMe)₈₀:* 10 g de NCA-GluOMe sont solubilisés dans un mélange de 150 ml de NMP à 60°C. 5 ml d'une solution de 0,91 g de benzylamine dans 50 ml de NMP sont ajoutés au monomère en une fois. Après 1 h, on ajoute 14,1 g de NCA-Leu solubilisé préalablement dans 20 ml de NMP. La polymérisation continue encore durant 3-4 h.

Un prélèvement du milieu réactionnel permet de connaître les caractérisations suivantes : Rendement 90 %. Composition par RMN 1H: (TFA-d) 66 % molaire GluOMe. Viscosité réduite (0,5% de TFA à 25°C) 0,4 dl/g. Masse Molaire par GPC : 20 000 g/mol.

### Exemple 2 - Aminolyse du poly(leucine)-bloc-(glutamate de méthyle) avec l'hydroxyéthylamine

A la solution de polymère dans la NMP préparé selon l'exemple 1, sont ajouté 10 g d'hydroxyéthylamine en une fois. Le milieu est porté et maintenu à 80°C pendant 2 jours. On ajoute ensuite 150 ml d'eau à la solution du polymère. Elle est ensuite purifiée par une étape de dialyse qui assure l'élimination de l'excès d'amine et la NMP. Les particules sont enfin isolées par lyophilisation.

Rendement quantitatif. RMN 1H (TFA-d) : 8% Methoxy résiduels (3.5ppm); 92% hydroxyéthylglutamine. Composition RMN 1H (TFA-d) : 34% Leu. Taille des particules (diffusion de la lumière mode QLS) : 100nm.

### Exemple 3 - Mise en évidence des nanoparticules par Diffusion de la Lumière (DDL) et par Microscopie Electronique à Transmission (TEM)

10 mg de particules du polymère 1 sont suspendus dans 10 ml d'eau ou une solution aqueuse de sel. Cette solution est ensuite introduite dans un granulomètre Coulter (ou diffractomètre laser). Les résultats de l'analyse de la granulométrie des différents produits testés sont présentés dans le tableau 1 suivant.

**Tableau 1 - Mesures de la taille des PV**

| Exemple | Polymère | Taille (nm) |
|---|---|---|
| 2 | POLY[(LEU)_{0.66}-BLOC-(GLN-N-HYDROXYETHYL)_{0,37}]ₓ | 100 |

### Exemple 4 : Test d'association des nanoparticules avec une protéine (l'insuline)

A partir d'une solution tampon phosphate isotonique de pH 7,4, on prépare une solution d'insuline humaine titrée à 1,4 mg/ml correspondant à 40 UI/ml. Dans 1 ml de cette solution d'insuline, on disperse 10 mg du **PV** préparé dans l'exemple 1. Après 15 heures d'incubation à température ambiante, l'insuline associée aux **PV** et l'insuline libre sont séparées par centrifugation (60 000 g, 1heure) et ultrafiltration (seuil de filtration 300 000 D). L'insuline libre récupérée dans le filtrat est dosée par CLHP ou par ELISA et l'on en déduit par différence la quantité d'insuline associée. La quantité d'insuline associée au **PV** est supérieure à 0,77 mg, ce qui représente plus de 55% du total de l'insuline engagée.

Le tableau suivant rassemble les résultats des mesures de taux d'association effectuées sur différents **PV.** Le taux d'association exprime le pourcentage d'insuline associée par rapport à l'insuline engagée dans une préparation titrée à 1.4 mg/ml d'insuline et 10 mg/ml de **PV.** Cette valeur est transformée en un taux de chargement qui exprime une formulation à 100% de fixation de la protéine, en mg d'insuline par 100 mg de **PV.**

**Tableau 2 - Mesures du taux d'association avec l'insuline pour un mélange 0.14 mg INSULINE/mg PV**

| Exemple | Polymère | Taux d'associatio n (%) | Taux de chargement mg/100mg PV |
|---|---|---|---|
| 2 | POLY[(LEU)_{0.66}-BLOC-(GLN-N-HYDROXYETHYL)_{0,37}]ₓ | 99 | 13.6 |

### Exemple 5 : Pharmacocinétique et pharmacodynamie des PV-charges avec l'insuline chez le chien sain à jeun

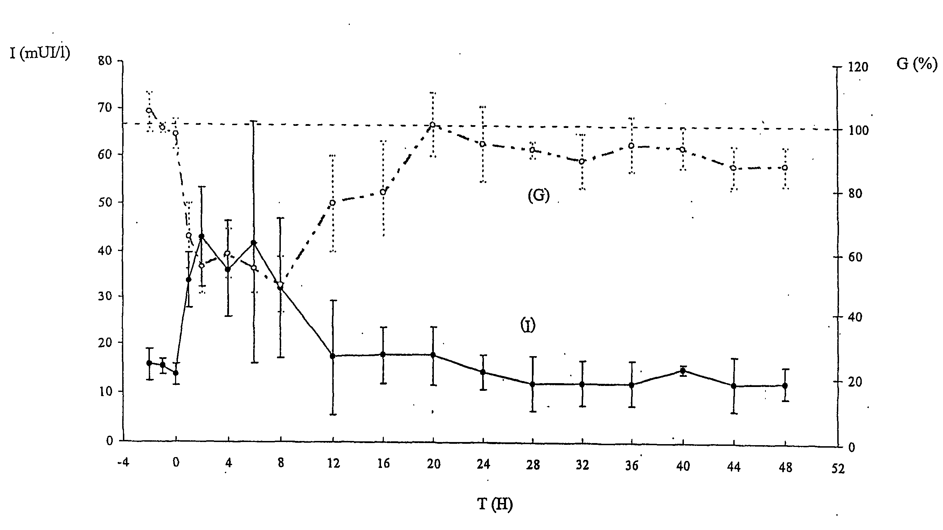

La préparation particules+insuline de l'exemple 4. a été injectée à des chiens, rendus diabétiques par pancréatectomie totale et à jeun de la veille au soir. L'administration à 11 heures du matin par voie sous cutanée thoracique de la préparation a été faite à la posologie de 0,5 UI/kg d'insuline par Kg de poids vif de l'animal. Le volume administré est compris entre 0,18 et 0,24 ml. Au temps -4, -2, 0, 1, 2, 4, 6, 8, 12, 16, 20, 24, 28, 32, 36, 40, 44 et 48 heures, 1 ml de sang sont prélevés par ponction jugulaire sous vide sur tube héparinate de sodium. 30 µl de sang total sont utilisés extemporanément pour mesure de la glycémie. Le tube est ensuite centrifugé, décanté et le plasma stocké à -20° C pour dosage de l'insuline. Les résultats présentés dans la figure 1 ci-après montrent un relarguage de l'insuline jusqu'à 12 heures (trait plein) et un effet hypoglycémiant important qui se prolonge jusqu'à 20 heures (trait discontinu) après l'injection.

**Tableau 3 - Mesures du temps d'action de l'insuline (effet hypoglycémiant) en présence de PV selon l'invention**

| Exemple | Polymère | Temps de retour au niveau basal (h) |
|---|---|---|
| | INSULINE SOLUBLE (SANS PV) | 1 |
| 2 | POLY[(LEU)_{0.66}-BLOC-(GLN-N-HYDROXYETHYL)_{0,37}]ₓ | 20H |

Cet exemple démontre la non dénaturation de l'insuline en présence de **PV** selon l'invention.

De plus, l'exemple 2 permet de mettre en évidence l'augmentation de la durée d'action de l'insuline par rapport à l'insuline non formulée, et donc, l'utilité des PV en tant que système retard pour la libération contrôlée de l'insuline. Elle montre également comment il est possible de maîtriser la durée d'action par la choix judicieuse du groupement hydrophobe.

### Exemple 6: Aminolyse du poly(Phénylalaniae)-bloc-(glutamate de méthyle) avec l'hydroxyéthylamine

A la solution de polymère dans la NMP préparé selon l'exemple 1 en partant des monomères NCAGluOMe et NCAPhénylalanine, sont ajouté 10 g d'hydroxyéthylamine en une fois. Le milieu est porté et maintenu à 80°C pendant 2 jours. On ajoute ensuite 150ml d'eau à la solution du polymère. Elle est ensuite purifié par une étape de dialyse qui assure l'élimination de l'excès d'amine et la NMP. Les particules sont enfin isolées par lyophilisation.

Rendement quantitatif. RMN 1H (TFA-d) : 6% Methoxy résiduels (3.5ppm); 94% hydroxyéthylglutamine. Composition RMN 1H (TFA-d): 34% Leu. Taille des particules (diffusion de la lumière mode QLS): 100 nm.

### Exemple 7: Aminolyse du poly(leucine-bloc-glutamate de benzyle) avec la 3-amino-propylène-1,2-glycol

A la solution de polymère dans la NMP préparée selon l'exemple 1, en partant des monomères NCAGluOBzl et NCALeu, sont ajoutés 10g de 3-amino-propylène-1,2-glycol en une fois. Le milieu est porté à 80°C pendant 2 jours. On ajoute ensuite 150 ml d'eau à la solution du polymère. Elle est ensuite purifiée par une étape de dialyse qui assure l'élimination de l'excès d'amine et du solvant. Les particules sont enfin isolées par lyophilisation. Rendement quantitatif. Taille des particules (QLS, selon l'exemple 3) : 100 nm.

### Exemple 8: Test d'association des nanoparticules avec une protéine (l'insuline)

Selon l'exemple 4, on met en oeuvre les particules isolées de l'exemple 6 et de l'insuline humaine pour obtenir un taux de chargement exprimé en mg d'insuline par 100mg de **PV.**

| Exemples | Polymère | Taux d'association | Taux de chargement en mg/100mg PV |
|---|---|---|---|
| 6 | POLY (PxE_{0.79}-BLOC-GLN-N-DIHYDROXYPROPYL_{0.21})ₓ | 99 | 8.5 |
| 7 | POLY (LEU_{0.66}-BLOC-GLN-N-DIHYDROXYPROPYL_{0.33})ₓ | 99 | 13.7 |

## Revendications

1. - Suspension colloïdale stable de particules structurées submicroniques susceptibles d'être utilisées, notamment pour la vectorisation de principe(s) actif(s) PA, ces particules étant des arrangements supramoléculaires individualisés (discrets) :
o à base de polyaminoacides (PAA) amphiphiles, linéaires, à enchaînements peptidiques et comprenant au moins deux types différents d'aminoacides récurrents hydrophiles AAI et hydrophobes AAO, les aminoacides de chaque type étant identiques ou différents entre eux ;
o aptes à associer en suspension colloïdale à l'état non dissous, au moins un PA et à libérer celui-ci, notamment in vivo, de manière prolongée et/ou retardée;
o et stables en phase aqueuse à pH compris entre 4 et 13 en l'absence de tensioactif(s);
**caractérisée** :
✔ en ce que les aminoacides récurrents hydrophiles AAI sont des aminoacides neutres hydrophiles AANI à l'exclusion de l'Asparagine;
✔ en ce que les aminoacides récurrents hydrophobes AAO sont des aminoacides neutres hydrophobes AANO;
✔ et en ce que les acides aminés récurrents de chaque type AANI et AANO sont identiques ou différents entre eux.

2. - Suspension selon la revendication 1, **caractérisée en ce que** les PAA constitutifs des particules sont des PAA "blocs", dans lesquels:
o le rapport molaire AANO/(AANI + AANO) est ≥ 6%,
o et la longueur absolue du bloc AANO est ≥ 5, de préférence ≥ 10, et plus préférentiellement ≥ 20.

3. - Suspension selon la revendication 1, **caractérisée en ce que** les PAA constitutifs des particules sont des PAA "copolymères statistiques" et **en ce que**:
• le rapport molaire AANI/(AANI + AANO) est ≥ 10%, et de préférence ≥ 20% et plus préférentiellement entre 30 et 70%,
• la masse molaire Mw ≥ 2000 Da.

4. - Suspension selon l'une quelconque des revendications 1 à 3, **caractérisée en ce que** l'(les)AANI hydrophile(s) est(sont) choisi(s) dans le groupe comprenant :
✔ les acides aminés neutres naturels, de préférence ceux choisis dans le groupe comprenant: sérine, thréonine, hydroxyproline, glutamine;
✔ les acides aminés neutres, rares ou synthétiques, de préférence ceux choisis dans le groupe comprenant : méthionine-S-oxyde, O-glycosidyl-sérine;
✔ les dérivés des acides aminés neutres, de préférence ceux choisis dans le groupe comprenant : N-hydroxyéthylglutamine, N-hydroxypropylasparagine, N-hydroxyéthyl-asparagine, N-hydroxypropylglutamine.

5. - Suspension selon l'une quelconque des revendications 1 à 4, **caractérisée en ce que** l'(les)AANO hydrophobe(s) est(sont) choisi(s) dans le groupe comprenant :
◆ les acides aminés neutres naturels, de préférence ceux choisis dans le groupe comprenant : Leu, Ile, Val, Ala, Pro, Phe ;
◆ les acides aminés neutres, rares ou synthétiques, de préférence ceux choisis dans le groupe comprenant : norleucine, norvaline ;
◆ les dérivés des acides aminés polaires, de préférence ceux choisis dans le groupe comprenant : glutamate de méthyle, glutamate d'éthyle, aspartate de benzyle, N-acétyllysine.

6. - Suspension selon l'une quelconque des revendications 1 à 5, **caractérisée en ce que** les particules de vectorisation (PV) qu'elle contient ont une de taille moyenne comprise entre 0,01 et 0,5 µm, de préférence entre 0,01 et 0,2 µm.

7. - Suspension colloïdale de particules, selon l'une quelconque des revendications 1 à 6, **caractérisée en ce qu'**elle est aqueuse et stable.

8. - Suspension selon l'une quelconque des revendications 1 à 7, **caractérisée en ce que** les particules comprennent au moins un principe actif.

9. - Solide pulvérulent **caractérisé en ce qu'**il est obtenu à partir de la suspension selon l'une quelconque des revendications 1 à 8.

10. - Procédé de préparation du solide pulvérulent selon la revendication 9 ou d'un solide pulvérulent obtenu à partir d'une suspension colloïdale stable de particules structurées submicroniques susceptibles d'être utilisées, notamment pour la vectorisation de principe(s) actif(s) **PA,** ces particules étant des arrangements supramoléculaires individualisés (discrets) :
o à base de polyaminoacides (PAA) amphiphiles, linéaires, à enchaînements peptidiques et comprenant au moins deux types différents d'aminoacides récurrents hydrophiles AAI et hydrophobes AAO, les aminoacides de chaque type étant identiques ou différents entre eux ;
o aptes à associer en suspension colloïdale à l'état non dissous, au moins un **PA** et à libérer celui-ci, notamment in vivo, de manière prolongée et/ou retardée ;
o et stables en phase aqueuse à pH compris entre 4 et 13 en l'absence de tensioactif(s);
dans laquelle:
✔ les aminoacides récurrents hydrophiles AAI sont au moins en partie constitués par de l'Asparagine,
✔ et les aminoacides récurrents hydrophobes AAO sont des aminoacides neutres hydrophobes AANO identiques ou différents entre eux,
**caractérisé en ce que** :
1) on réalise une copolymérisation de monomères formés par des anhydrides de N-CarboxyAminoacides (NCA) d'au moins deux types différents,
◆ d'une part, des monomères NCA de départ comprenant des NCA-Glu-OR et/ou des NCA-Asp-OR, et/ou des NCA-AANI,
◆ et d'autre part, des NCA-AANO, en présence:
- d'au moins un solvant polaire non aromatique, de préférence choisi dans le groupe comprenant: la N-MéthylPyrrolidone (NMP), le DiMéthylFormamide (DMF), le DiMéthylsulfOxyde (DMSO), le DiMéthylAcétamide (DMAc), la pyrrolidone ; la NMP étant plus particulièrement préférées ;
- et éventuellement d'au moins un co-solvant sélectionné parmi les solvants aprotiques (de préférence le dioxanne-1,4) et/ou les solvants protiques (de préférence la pyrrolidone) et/ou l'eau et/ou les alcools, le méthanol étant particulièrement préféré ;
2) dans le cas où les monomères NCA de départ sont des NCA-Glu-OR et/ou des NCA-Asp-OR (R = alkyle), on met en oeuvre une aminolyse qui consiste à mettre en présence le copolymère obtenu à l'étape 1 avec une phase aqueuse comprenant au moins une amine et qui permet la transformation de Glu-OR en Gln et de Asp-OR en Asn ;
3) éventuellement on dialyse le milieu réactionnel pour purifier la suspension aqueuse de particules structurées ;
4) éventuellement on concentre cette suspension de l'étape 3 ;
5) on élimine le milieu liquide pour recueillir le solide pulvérulent comprenant les particules.

11. - Procédé de préparation de la suspension selon l'une quelconque des revendications 1 à 8, **caractérisé en ce que** l'on met en présence d'un milieu aqueux, le solide pulvérulent selon la revendication 9 et/ou le solide pulvérulent obtenu par le procédé selon la revendication 10.

12. - Procédé de préparation de la suspension selon la revendication 8, ou d'une suspension colloïdale stable de particules structurées submicroniques chargées en principe(s) actif(s) **PA,** ces particules étant des arrangements supramoléculaires individualisés (discrets) :
o à base de polyaminoacides (PAA) amphiphiles, linéaires, à enchaînements peptidiques et comprenant au moins deux types différents d'aminoacides récurrents hydrophiles AAI et hydrophobes AAO, les aminoacides de chaque type étant identiques ou différents entre eux ;
o aptes à associer en suspension colloïdale à l'état non dissous, au moins un **PA** et à libérer celui-ci, notamment in vivo, de manière prolongée et/ou retardée ;
o et stables en phase aqueuse à pH compris entre 4 et 13 en l'absence de tensioactif(s) ;
dans laquelle:
✔ les aminoacides récurrents hydrophiles AAI sont au moins en partie constitués par de l'Asparagine,
✔ et les aminoacides récurrents hydrophobes AAO sont des aminoacides neutres hydrophobes AANO identiques ou différents entre eux,
**caractérisé en ce que** l'on effectue l'association du PA aux particules, par mise en présence d'une phase liquide contenant le PA avec la suspension colloïdale de particules et/ou le solide pulvérulent obtenu par le procédé selon la revendication 10.

13. - Procédé de préparation de la suspension selon la revendication 8 ou d'une suspension colloïdale stable de particules structurées submicroniques chargées en principe(s) actif(s) **PA,** ces particules étant des arrangements supramoléculaires individualisés (discrets) :
o à base de polyaminoacides (PAA) amphiphiles, linéaires, à enchaînements peptidiques et comprenant au moins deux types différents d'aminoacides récurrents hydrophiles AAI et hydrophobes AAO, les aminoacides de chaque type étant identiques ou différents entre eux ;
o aptes à associer en suspension colloïdale à l'état non dissous, au moins un **PA** et à libérer celui-ci, notamment in vivo, de manière prolongée et/ou retardée ;
o et stables en phase aqueuse à pH compris entre 4 et 13 en l'absence de tensioactif(s) ;
dans laquelle:
✔ les aminoacides récurrents hydrophiles AAI sont au moins en partie constitués par de l'Asparagine,
✔ et les aminoacides récurrents hydrophobes AAO sont des aminoacides neutres hydrophobes AANO identiques ou différents entre eux,
**caractérisé en ce que** l'on effectue l'association du PA aux particules par mise en présence d'un PA à l'état solide avec la suspension colloïdale de particules.

14. - Procédé de préparation de la suspension selon la revendication 8, ou d'une suspension colloïdale stable de particules structurées submicroniques chargées en principe(s) actif(s) **PA,** ces particules étant des arrangements supramoléculaires individualisés (discrets) :
o à base de polyaminoacides (PAA) amphiphiles, linéaires, à enchaînements peptidiques et comprenant au moins deux types différents d'aminoacides récurrents hydrophiles AAI et hydrophobes AAO, les aminoacides de chaque type étant identiques ou différents entre eux ;
o aptes à associer en suspension colloïdale à l'état non dissous, au moins un **PA** et à libérer celui-ci, notamment in vivo, de manière prolongée et/ou retardée ;
o et stables en phase aqueuse à pH compris entre 4 et 13 en l'absence de tensioactif(s) ;
dans laquelle:
✔ les aminoacides récurrents hydrophiles AAI sont au moins en partie constitués par de l'Asparagine,
✔ et les aminoacides récurrents hydrophobes AAO sont des aminoacides neutres hydrophobes AANO identiques ou différents entre eux,
**caractérisé en ce que** l'on met en présence le solide pulvérulent selon la revendication 9 et/ou le solide pulvérulent obtenu par le procédé selon la revendication 10, avec une phase liquide contenant le PA.

15. - Procédé de préparation de la suspension selon la revendication 9, **caractérisé en ce que** l'on met en présence le solide pulvérulent selon la revendication 9 et/ou le solide pulvérulent obtenu par le procédé selon la revendication 10, avec le PA sous forme solide et **en ce que** l'on disperse ce mélange de solides dans une phase liquide, de préférence une solution aqueuse.

16. - Produits intermédiaires du procédé selon la revendication 10, **caractérisés en ce qu'**ils sont constitués par des copolymères PAA précurseurs de particules.

17. - Suspension selon la revendication 9 et/ou obtenue par le procédé selon l'une quelconque des revendications 12 à 15 et/ou solide pulvérulent selon la revendication 9, et/ou obtenu par le procédé selon la revendication 10, comprenant un moins un principe actif choisi, de préférence, parmi:
• les vaccins ;
• les protéines et/ou les peptides, parmi lesquels les plus préférentiellement retenus sont : les hémoglobines, les cytochromes, les albumines, les interférons, les antigènes, les anticorps, l'érythropoïétine, l'insuline, les hormones de croissance, les facteurs VIII et IX, les interleukines ou leurs mélanges, les facteurs stimulants de l'hématopoïèse ;
• les polysaccharides, l'héparine étant plus particulièrement sélectionnée ;
• les acides nucléiques et, préférablement, les oligonucléotides d'ARN et/ou d'ADN ;
• des molécules non peptido-protéiques appartenant à diverses classes de chimiothérapie anti-cancéreuses et, en particulier, les anthracyclines et les taxoïdes, et leurs mélanges.

18. - Suspension selon la revendication 7 et/ou suspension obtenue par le procédé selon l'une quelconque des revendications 12 à 15, et/ou solide pulvérulent selon la revendication 9 ou obtenu par le procédé selon la revendication 10, comprenant au moins un principe actif nutritionnel, phytosanitaire ou cosmétique.

19. - Spécialité pharmaceutique, nutritionnelle, phytosanitaire ou cosmétique, **caractérisée en ce qu'**elle comporte une suspension et/ou du solide pulvérulent selon la revendication 17 ou 18.

20. - Utilisation d'une suspension colloïdale stable de particules structurées submicroniques chargées en principe(s) actif(s) **PA,** ces particules étant des arrangements supramoléculaires individualisés (discrets) :
o à base de polyaminoacides (PAA) amphiphiles, linéaires, à enchaînements peptidiques et comprenant au moins deux types différents d'aminoacides récurrents hydrophiles AAI et hydrophobes AAO, les aminoacides de chaque type étant identiques ou différents entre eux ;
o aptes à associer en suspension colloïdale à l'état non dissous, au moins un **PA** et à libérer celui-ci, notamment in vivo, de manière prolongée et/ou retardée ;
o et stables en phase aqueuse à pH compris entre 4 et 13 en l'absence de tensioactif(s);
dans laquelle:
✔ les aminoacides récurrents hydrophiles AAI sont au moins en partie constitués par de l'Asparagine,
✔ et les aminoacides récurrents hydrophobes AAO sont des aminoacides neutres hydrophobes AANO identiques ou différents entre eux ;
pour la préparation d'une suspension aqueuse ou d'un solide pulvérulent, chargé en au moins un PA et tel que défini dans les revendications précédentes.

## Claims

1. Stable colloidal suspension of submicronic structured particles which can be used, in particular for carrying active principle(s) **PA,** these particles being individualized (discrete) supramolecular arrangements:
o based on linear, amphiphilic polyamino acids (PAA), with peptide linkages and comprising at least two different types of recurring amino acids, hydrophilic AAI and hydrophobic AAO, the amino acids of each type being mutually identical or different;
o capable of combining in colloidal suspension in the nondissolved state, at least one **PA** and of releasing it, in particular in vivo, in a prolonged and/or delayed manner;
o and stable in aqueous phase at a pH between 4 and 13 in the absence of surfactant(s);
**characterized**:
✔ in that the hydrophilic recurring amino acids AAI are hydrophilic neutral amino acids AANI excluding asparagine;
✔ in that the hydrophobic recurring amino acids AAO are hydrophobic neutral amino acids AANO;
✔ and in that the recurring amino acids of each type, AANI and AANO, are mutually identical or different.

2. Suspension according to Claim 1, **characterized in that** the constituent PAAs of the particles are "block" PAAs, in which:
o the AANO/(AANI + AANO) molar ratio is ≥ 6%,
o and the absolute length of the AANO block is ≥ 5, preferably ≥ 10, and more preferably ≥ 20.

3. Suspension according to Claim 1, **characterized in that** the constituent PAAs of the particles are "random copolymer" known as PAAs and **in that**:
• the AANI/(AANI + AANO) molar ratio is ≥ 10%, and preferably ≥ 20% and more preferably between 30 and 70%,
• the molar mass Mw is ≥ 2000 Da.

4. Suspension according to any one of Claims 1 to 3, **characterized in that** the hydrophilic AANI(s) is (are) chosen from the group comprising:
✔ natural neutral amino acids, preferably those chosen from the group comprising: serine, threonine, hydroxyproline, glutamine;
✔ rare or synthetic neutral amino acids, preferably those chosen from the group comprising: methionine S-oxide, O-glycosidyl-serine;
✔ derivatives of neutral amino acids, preferably those chosen from the group comprising: N-hydroxyethylglutamine, N-hydroxypropylasparagine, N-hydroxyethyl-asparagine, N-hydroxypropylglutamine.

5. Suspension according to any one of Claims 1 to 4, **characterized in that** the hydrophobic AANO(s) is (are) chosen from the group comprising:
◆ natural neutral amino acids, preferably those chosen from the group comprising: Leu, Ile, Val, Ala, Pro, Phe;
◆ rare or synthetic neutral amino acids, preferably those chosen from the group comprising: norleucine, norvaline;
◆ derivatives of polar amino acids, preferably those chosen from the group comprising: methyl glutamate, ethyl glutamate, benzyl aspartate, N-acetyllysine.

6. Suspension according to any one of Claims 1 to 5, **characterized in that** the vector particles (PV) which it contains have a mean size between 0.01 and 0.5 µm, preferably between 0.01 and 0.2 µm.

7. Colloidal suspension of particles according to any one of Claims 1 to 6, **characterized in that** it is aqueous and stable.

8. Suspension according to any one of Claims 1 to 7, **characterized in that** the particles comprise at least one active principle.

9. Pulverulent solid, **characterized in that** it is obtained from the suspension according to any one of Claims 1 to 8.

10. Method for preparing the pulverulent solid according to Claim 9 or a pulverulent solid obtained from a stable colloidal suspension of submicronic structured particles which can be used, in particular for carrying active principle(s) **PA,** these particles being individualized (discrete) supramolecular arrangements:
o based on linear, amphiphilic polyamino acids (PAA), with peptide linkages and comprising at least two different types of recurring amino acids, hydrophilic AAI and hydrophobic AAO, the amino acids of each type being mutually identical or different;
o capable of combining in colloidal suspension in the nondissolved state, at least one **PA** and of releasing it, in particular in vivo, in a prolonged and/or delayed manner;
o and stable in aqueous phase at a pH between 4 and 13 in the absence of surfactant(s);
in which:
✔ the hydrophilic recurring amino acids AAI at least partly consist of asparagine;
✔ and the hydrophobic recurring amino acids AAO are hydrophobic neutral amino acids AANO which are mutually identical or different;
**characterized in that**:
1) copolymerization of monomers consisting of anhydrides of N-carboxyamino acids (NCA) of at least two different types is carried out,
◆ on the one hand, starting NCA monomers comprising NCA-Glu-ORs and/or NCA-Asp-ORs, and/or NCA-AANIs,
◆ and on the other hand, NCA-AANOs, in the presence:
- of at least one nonaromatic polar solvent, preferably chosen from the group comprising: N-MethylPyrrolidone (NMP), DiMethylFormamide (DMF), DiMethylSulphOxide (DMSO), DiMethylAcetamide (DMAc), pyrrolidone, NMP being more particularly preferred;
- and optionally of at least one co-solvent selected from aprotic solvents (preferably 1,4-dioxane) and/or protic solvents (preferably pyrrolidone) and/or water and/or alcohols, methanol being particularly preferred;
2) in the case where the starting NCA monomers are NCA-Glu-ORs and/or NCA-Asp-ORs (R = alkyl), aminolysis is carried out which consists in bringing the copolymer obtained in step 1 into contact with an aqueous phase comprising at least one amine and which allows the conversion of Glu-OR to Gln and of Asp-OR to Asn;
3) optionally the reaction medium is dialysed in order to purify the aqueous suspension of structured particles;
4) optionally this suspension of step 3 is concentrated;
5) the liquid medium is removed in order to collect the pulverulent solid comprising the particles.

11. Method for preparing the suspension according to any one of Claims 1 to 8, **characterized in that**, the pulverulent solid according to Claim 9 and/or the pulverulent solid obtained by the method according to Claim 10 is (are) brought into contact with an aqueous medium.

12. Method for preparing the suspension according to Claim 8, or a stable colloidal suspension of submicronic structured particles loaded with active principle(s) **PA,** these particles being individualized (discrete) supramolecular arrangements:
o based on linear, amphiphilic polyamino acids (PAA), with peptide linkages and comprising at least two different types of recurring amino acids, hydrophilic AAI and hydrophobic AAO, the amino acids of each type being mutually identical or different;
o capable of combining in colloidal suspension in the nondissolved state, at least one **PA** and of releasing it, in particular in vivo, in a prolonged and/or delayed manner;
o and stable in aqueous phase at a pH between 4 and 13 in the absence of surfactant(s);
in which:
✔ the hydrophilic recurring amino acids AAI at least partly consist of asparagine;
✔ and the hydrophobic recurring amino acids AAO are hydrophobic neutral amino acids AANO which are mutually identical or different;
**characterized in that** the combination of the PA with the particles is carried out by bringing a liquid phase containing the PA into contact with the colloidal suspension of particles and/or the pulverulent solid obtained by the method according to Claim 10.

13. Method for preparing the suspension according to Claim 8, or a stable colloidal suspension of submicronic structured particles loaded with active principle(s) **PA,** these particles being individualized (discrete) supramolecular arrangements:
o based on linear, amphiphilic polyamino acids (PAA), with peptide linkages and comprising at least two different types of recurring amino acids, hydrophilic AAI and hydrophobic AAO, the amino acids of each type being mutually identical or different;
o capable of combining in colloidal suspension in the nondissolved state, at least one **PA** and of releasing it, in particular in vivo, in a prolonged and/or delayed manner;
o and stable in aqueous phase at a pH between 4 and 13 in the absence of surfactant(s);
in which:
✔ the hydrophilic recurring amino acids AAI at least partly consist of asparagine;
✔ and the hydrophobic recurring amino acids AAO are hydrophobic neutral amino acids AANO which are mutually identical or different;
**characterized in that** the combination of the PA with the particles is carried out by bringing a PA in the solid state into contact with the colloidal suspension of particles.

14. Method for preparing the suspension according to Claim 8, or a stable colloidal suspension of submicronic structured particles loaded with active principle(s) **PA,** these particles being individualized (discrete) supramolecular arrangements:
o based on linear, amphiphilic polyamino acids (PAA), with peptide linkages and comprising at least two different types of recurring amino acids, hydrophilic AAI and hydrophobic AAO, the amino acids of each type being mutually identical or different;
o capable of combining in colloidal suspension in the nondissolved state, at least one **PA** and of releasing it, in particular in vivo, in a prolonged and/or delayed manner;
o and stable in aqueous phase at a pH between 4 and 13 in the absence of surfactant(s);
in which:
✔ the hydrophilic recurring amino acids AAI at least partly consist of asparagine;
✔ and the hydrophobic recurring amino acids AAO are hydrophobic neutral amino acids AANO which are mutually identical or different;
**characterized in that** the pulverulent solid according to Claim 9 and/or the pulverulent solid obtained by the method according to Claim 10 is (are) brought into contact with a liquid phase containing the PA.

15. Method for preparing the suspension according to Claim 9, **characterized in that** the pulverulent solid according to Claim 9 and/or the pulverulent solid obtained by the method according to Claim 10 is (are) brought into contact with the PA in solid form and **in that** this mixture of solids is dispersed in a liquid phase, preferably an aqueous solution.

16. Intermediate products of the method according to Claim 10, **characterized in that** they consist of PAA copolymers which are precursors of particles.

17. Suspension according to Claim 9 and/or obtained by the method according to any one of Claims 12 to 15 and/or pulverulent solid according to Claim 9 and/or obtained by the method according to Claim 10, comprising at least one active principle preferably chosen from:
• vaccines;
• proteins and/or peptides, among which those most preferably selected are: haemoglobins, cytochromes, albumins, interferons, antigens, antibodies, erythropoietin, insulin, growth hormones, factors VIII and IX, interleukins or mixtures thereof, haematopoiesis-stimulating factors;
• polysaccharides, heparin being more particularly selected;
• nucleic acids and, preferably, RNA and/or DNA oligonucleotides;
• non-peptido-protein molecules belonging to the various anticancer chemotherapy classes and, in particular, anthracyclins and taxoids, and mixtures thereof.

18. Suspension according to Claim 7 and/or suspension obtained by the method according to any one of Claims 12 to 15, and/or pulverulent solid according to Claim 9 or obtained by the method according to Claim 10, comprising at least one nutritional, plant-protection or cosmetic active principle.

19. Pharmaceutical, nutritional, plant-protection or cosmetic proprietary product, **characterized in that** it comprises a suspension and/or pulverulent solid according to Claim 17 or 18.

20. Use of a stable colloidal suspension of submicronic structured particles loaded with active principle(s) **PA,** these particles being individualized (discrete) supramolecular arrangements:
o based on linear, amphiphilic polyamino acids (PAA), with peptide linkages and comprising at least two different types of recurring amino acids, hydrophilic AAI and hydrophobic AAO, the amino acids of each type being mutually identical or different;
o capable of combining in colloidal suspension in the nondissolved state, at least one **PA** and of releasing it, in particular in vivo, in a prolonged and/or delayed manner;
o and stable in aqueous phase at a pH between 4 and 13 in the absence of surfactant(s);
in which:
✔ the hydrophilic recurring amino acids AAI at least partly consist of asparagine;
✔ and the hydrophobic recurring amino acids AAO are hydrophobic neutral amino acids AANO which are mutually identical or different;
for the preparation of an aqueous suspension or a pulverulent solid, loaded with at least one PA and as defined in the preceding claims.

## Patentansprüche

1. Kolloidale stabile Suspension strukturierter submikronischer Partikel, die sich insbesondere zur Verwendung für die Vektorisierung eines oder mehrerer Wirkprinzipien (PA) eignen, wobei diese Partikel in supramolekularen vereinzelten (diskreten) Anordnungen vorliegen:
- auf Basis amphiphiler, linearer Polyaminosäuren (PAA) mit peptidischen Ketten, die mindestens zwei verschiedene Typen wiederkehrender hydrophiler Aminosäuren AAI und hydrophober Aminosäuren AAO umfassen, wobei die Aminosäuren jeden Typs gleich oder verschieden voneinander sind;
- wobei sie sich dazu eignen, in kolloidaler Suspension in nicht gelöstem Zustand mindestens einen PA anzulagern und diesen, insbesondere in vivo, verlängert und/oder verzögert wieder freizusetzen; und
- wobei sie in wässriger Phase bei einem pH-Wert von 4 bis 13 in Abwesenheit oberflächenaktiver Mittel stabil sind,
**dadurch gekennzeichnet, dass**:
- die wiederkehrenden hydrophilen Aminosäuren AAI neutrale hydrophile Aminosäuren AANI sind, ausgenommen Asparagin;
- die wiederkehrenden hydrophoben Aminosäuren AAO neutrale hydrophobe Aminosäuren AANO sind;
- und die aminierten wiederkehrenden Säuren jeden Typs AANI und AANO gleich oder verschieden voneinander sind.

2. Suspension gemäß Anspruch 1, **dadurch gekennzeichnet, dass** die PAA-Bestandteile der Partikel PAA-"Blöcke" sind, in denen:
- das Molverhältnis AANO/(AANI + AANO) ≥ 6 %
- und die absolute Länge des AANO-Blocks ≥ 5, vorzugsweise ≥ 10 und bevorzugter ≥ 20 betragen.

3. Suspension gemäß Anspruch 1, **dadurch gekennzeichnet, dass** die PAA-Bestandteile der Partikel "statistische Copolymere" von PAA sind und dass:
- das Molverhältnis AANI/(AANI + AANO) ≥ 10 %, vorzugsweise ≥ 20 % und bevorzugter 30 bis 70 % und
- die Molmasse Mw ≥ 2000 Da betragen.

4. Suspension gemäß einem der Ansprüche 1 bis 3, **dadurch gekennzeichnet, dass** die hydrophile(n) AANI aus der Gruppe ausgewählt sind, umfassend:
- aminierte neutrale natürliche Säuren, vorzugsweise diejenigen, ausgewählt aus der Gruppe, umfassend: Serin, Threonin, Hydroxyprolin, Glutamin;
- aminierte neutrale, seltene oder synthetische Säuren, vorzugsweise diejenigen, ausgewählt aus der Gruppe, umfassend: Methionin-S-oxid, O-Glycosidylserin;
- Derivate aminierter neutraler Säuren, vorzugsweise diejenigen, ausgewählt aus der Gruppe, umfassend: N-Hydroxyethylglutamin, N-Hydroxypropylasparagin, N-Hydroxyethylasparagin, N-Hydroxypropylglutamin.

5. Suspension gemäß einem der Ansprüche 1 bis 4, **dadurch gekennzeichnet, dass** die hydrophobe(n) AANO aus der Gruppe ausgewählt sind, umfassend:
- aminierte neutrale natürliche Säuren, vorzugsweise diejenigen, ausgewählt aus der Gruppe, umfassend: Leu, Ile, Val, Ala, Pro, Phe;
- aminierte neutrale, seltene oder synthetische Säuren, vorzugsweise diejenigen, ausgewählt aus der Gruppe, umfassend: Norleucin, Norvalin;
- Derivate aminierter polarer Säuren, vorzugsweise diejenigen, ausgewählt aus der Gruppe, umfassend: Methylglutamat, Ethylglutamat, Benzylaspariginat, N-Acetyllysin.

6. Suspension gemäß einem der Ansprüche 1 bis 5, **dadurch gekennzeichnet, dass** die Vektorisierungspartikel (PV), die darin enthalten sind, eine mittlere Größe von 0,01 bis 0,5 und vorzugsweise von 0,01 bis 0,2 µm aufweisen.

7. Kolloidale Partikelsuspension gemäß einem der Ansprüche 1 bis 6, **dadurch gekennzeichnet, dass** sie wässrig und stabil ist.

8. Suspension gemäß einem der Ansprüche 1 bis 7, **dadurch gekennzeichnet, dass** die Partikel mindestens ein Wirkprinzip umfassen.

9. Pulverförmiger Feststoff, **dadurch gekennzeichnet, dass** er aus der Suspension gemäß einem der Ansprüche 1 bis 8 erhältlich ist.

10. Verfahren zur Herstellung des pulverförmigen Feststoffs gemäß Anspruch 9 oder eines pulverförmigen Feststoffs, erhältlich aus einer kolloidalen stabilen Suspension strukturierter submikronischer Partikel, die sich insbesondere zur Verwendung für die Vektorisierung von Wirkprinzipien PA eignen und in supramolekularen vereinzelten (diskreten) Anordnungen vorliegen:
- auf Basis amphiphiler, linearer Polyaminosäuren (PAA) mit peptidischen Ketten und mindestens zwei unterschiedlichen Typen wiederkehrender hydrophiler Aminosäuren AAI und hydrophober Aminosäuren AAO, wobei die Aminosäuren jeden Typs gleich oder verschieden voneinander sind;
- wobei sie sich dazu eignen, in kolloidaler Suspension im nicht gelösten Zustand mindestens ein PA anzulagern und dieses, insbesondere in vivo, verlängert und/oder verzögert erneut freizusetzen;
- und wobei sie in wässriger Phase bei einem pH-Wert von 4 bis 13 in der Abwesenheit oberflächenaktiver Mittel stabil sind,
in denen:
- die wiederkehrenden hydrophilen Aminosäuren AAI mindestens teilweise aus Asparagin zusammengesetzt
- und die wiederkehrenden hydrophoben Aminosäuren AAO neutrale hydrophobe Aminosäuren AANO sind, die gleich oder verschieden voneinander sind,
**dadurch gekennzeichnet, dass**:
1) man eine Copolymerisation von Monomeren aus N-Carboxyaminosäureanhydriden (NCA) aus mindestens 2 unterschiedlichen Typen
- einerseits aus NCA-Ausgangsmonomeren, die NCA-Glu-OR und/oder NCA-Asp-OR und/oder NCA-AANI umfassen,
- und andererseits aus NCA-AANO in der Gegenwart:
- mindestens eines polaren nicht aromatischen Lösungsmittels, das vorzugsweise aus der Gruppe ausgewählt ist, umfassend: N-Methylpyrrolidon (NMP), Dimethylformamid (DMF), Dimethylsulfoxid (DMSO), Dimethylacetamid (DMAc), Pyrrolidon, wobei NMP am meisten bevorzugt ist;
- und gegebenenfalls mindestens eines Co-Lösungsmittels durchführt, das aus aprotischen Lösungsmitteln (vorzugsweise aus 1,4-Dioxan) und/oder protischen Lösungsmitteln (vorzugsweise aus Pyrrolidon) und/oder aus Wasser und/oder aus Alkoholen ausgewählt ist, wobei Methanol besonders bevorzugt ist;
2) dass man im Fall, dass die NCA-Ausgangsmonomeren NCA-Glu-OR und/oder NCA-Asp-OR (R = Alkyl) sind, eine Aminolyse durchführt, wobei man das in Stufe 1) erhaltene Copolymer mit einer wässrigen Phase zusammenbringt, die mindestens ein Amin umfasst und die Überführung von Glu-OR in Gln und von Asp-OR in Asn ermöglicht;
3) man gegebenenfalls das Reaktionsmilieu dialysiert, um die wässrige Suspension strukturierter Partikel zu reinigen,
4) man gegebenenfalls diese Suspension aus der Stufe 3) einengt; und dass
5) man das flüssige Milieu beseitigt, um den pulverförmigen Feststoff zu gewinnen, der die Partikel umfasst.

11. Verfahren zur Herstellung der Suspension gemäß einem der Ansprüche 1 bis 8, **dadurch gekennzeichnet, dass** man den pulverförmigen Feststoff gemäß Anspruch 9 und/oder den mit dem Verfahren gemäß Anspruch 10 erhaltenen pulverförmigen Feststoff mit einem wässrigen Milieu zusammenbringt.

12. Verfahren zur Herstellung der Suspension gemäß Anspruch 8 oder einer kolloidalen stabilen Suspension strukturierter submikronischer Partikel, die mit einem oder mehreren Wirkprinzipien PA beaufschlagt sind, wobei diese Partikel in supramolekularen vereinzelten (diskreten) Anordnungen vorliegen:
- auf Basis amphiphiler, linearer Polyaminosäuren (PAA) mit peptidischen Ketten mit mindestens 2 unterschiedlichen Typen wiederkehrender hydrophiler Aminosäuren AAI und hydrophober Aminosäuren AAO, wobei die Aminosäuren jeden Typs gleich oder verschieden voneinander sind;
- wobei sie sich dazu eignen, in kolloidaler Suspension im nicht gelösten Zustand mindestens ein PA anzulagern und dieses, insbesondere in vivo, verlängert und/oder verzögert erneut freizusetzen;
- und wobei sie in wässriger Phase bei einem pH-Wert von 4 bis 13 in Abwesenheit oberflächenaktiver Mittel stabil sind,
in denen:
die wiederkehrenden hydrophilen Aminosäuren AAI zumindest teilweise aus Asparagin zusammengesetzt
und die wiederkehrenden hydrophoben Aminosäuren AAO neutrale hydrophobe Aminosäuren AANO sind, die gleich oder verschieden voneinander sind,
**dadurch gekennzeichnet, dass** man die Anlagerung des PA an die Partikel **dadurch** bewerkstelligt, dass man eine flüssige Phase, die das PA enthält, mit der kolloidalen Partikel-Suspension und/oder mit dem durch das Verfahren gemäß Anspruch 10 erhaltenen pulverförmigen Feststoff zusammenbringt.

13. Verfahren zur Herstellung der Suspension gemäß Anspruch 8 oder einer kolloidalen stabilen Suspension strukturierter submikronischer Partikel, die mit einem oder mehreren Wirkprinzipien PA beaufschlagt sind, wobei diese Partikel in supramolekularen vereinzelten (diskreten) Partikeln vorliegen:
- auf Basis amphiphiler, linearer Polyaminosäuren (PAA) mit peptidischen Ketten mit mindestens 2 unterschiedlichen Typen wiederkehrender hydrophiler Aminosäuren AAI und hydrophober Aminosäuren AAO, wobei die Aminosäuren jeden Typs gleich oder verschieden voneinander sind;
- wobei sie sich dazu eignen, in kolloidaler Suspension im nicht gelösten Zustand mindestens ein PA anzulagern und dieses, insbesondere in vivo, verlängert und/oder verzögert erneut freizusetzen;
- und wobei sie in wässriger Phase bei einem pH-Wert von 4 bis 13 in Abwesenheit oberflächenaktiver Mittel stabil sind,
in denen:
die wiederkehrenden hydrophilen Aminosäuren AAI zumindest teilweise aus Asparagin zusammengesetzt und
die wiederkehrenden hydrophoben Aminosäuren AAO neutrale hydrophobe Aminosäuren AANO sind, die gleich oder verschieden voneinander sind,
**dadurch gekennzeichnet, dass** man die Anlagerung des PAA an die Partikel **dadurch** bewerkstelligt, dass man ein PA im Zustand eines Feststoffs mit der kolloidalen Partikel-Suspension zusammenbringt.

14. Verfahren zur Herstellung der Suspension gemäß Anspruch 8 oder einer kolloidalen stabilen Suspension strukturierter submikronischer Partikel, die mit einem oder mehreren Wirkprinzipien PA beaufschlagt sind, wobei diese Partikel in supramolekularen vereinzelten (diskreten) Partikeln vorliegen:
- auf Basis amphiphiler, linearer Polyaminosäuren (PAA) mit peptidischen Ketten mit mindestens 2 unterschiedlichen Typen wiederkehrender hydrophiler Aminosäuren AAI und hydrophober Aminosäuren AAO, wobei die Aminosäuren jeden Typs gleich oder verschieden voneinander sind;
- wobei sie sich dazu eignen, in kolloidaler Suspension im nicht gelösten Zustand mindestens ein PA anzulagern und dieses, insbesondere in vivo, verlängert und/oder verzögert erneut freizusetzen;
- und wobei sie in wässriger Phase bei einem pH-Wert von 4 bis 13 in Abwesenheit oberflächenaktiver Mittel stabil sind,
in denen:
die wiederkehrenden hydrophilen Aminosäuren AAI zumindest teilweise aus Asparagin zusammengesetzt
und die wiederkehrenden hydrophoben Aminosäuren AAO neutrale phydrophobe Aminosäuren AANO sind, die gleich oder verschieden voneinander sind,
**dadurch gekennzeichnet, dass** man den pulverförmigen Feststoff gemäß Anspruch 9 und/oder den mit dem Verfahren gemäß Anspruch 10 erhaltenen pulverförmigen Feststoff mit einer flüssigen Phase zusammenbringt, die das PA enthält.

15. Verfahren zur Herstellung der Suspension gemäß Anspruch 9, **dadurch gekennzeichnet, dass** man den pulverförmigen Feststoff gemäß Anspruch 9 und/oder den mit dem Verfahren gemäß Anspruch 10 erhaltenen pulverförmigen Feststoff mit dem PA in Form eines Feststoffs zusammenbringt und man diese Mischung aus Feststoffen in einer flüssigen Phase, vorzugsweise in einer wässrigen Lösung, dispergiert.

16. Zwischenproduktverbindungen des Verfahrens gemäß Anspruch 10, **dadurch gekennzeichnet, dass** sie aus den Partikelvorstufen der PAA-Copolymeren zusammengesetzt sind.

17. Suspension gemäß Anspruch 9 und/oder mit dem Verfahren gemäß einem der Ansprüche 12 bis 15 erhaltene Suspension und/oder pulverförmiger Feststoff gemäß Anspruch 9 und/oder mit dem Verfahren gemäß Anspruch 10 erhaltener pulverförmiger Feststoff, welche mindestens ein Wirkprinzip umfassen, das vorzugsweise ausgewählt ist aus:
- Impfstoffen;
- Proteinen und/oder Peptiden, unter denen die am meisten bevorzugten umfassen: Hämoglobine, Cytochrome, Albumine, Interferone, Antigene, Antikörper, Erythropoietin, Insulin, Wachstumshormone, die Faktoren VIII und IX, Interleucine oder deren Mischungen, Hämatopoiese-Stimulierfaktoren;
- Polysacchariden, wobei Heparin am meisten bevorzugt ist;
- Nucleinsäuren und vorzugsweise RNA- und/oder DNA-Oligonucleotiden;
- Nicht-peptido-proteinischen Molekülen, die zu diversen Klassen einer Antikrebs-Chemotherapie gehören, und insbesondere aus Anthracyclinen und Taxoiden sowie aus deren Mischungen.

18. Suspension gemäß Anspruch 7 und/oder mit dem Verfahren gemäß einem der Ansprüche 12 bis 15 erhaltene Suspension und/oder pulverförmiger Feststoff gemäß Anspruch 9 oder mit dem Verfahren gemäß Anspruch 10 erhaltener pulverförmiger Feststoff, welche mindestens ein Nahrungsmittel-, phytosanitäres oder kosmetisches Wirkprinzip umfassen.

19. Pharmazeutisches, Nahrungsmittel-, phytosanitäres oder kosmetisches Spezialprodukt, **dadurch gekennzeichnet, dass** es eine Suspension und/oder pulverförmigen Feststoff gemäß Anspruch 17 oder 18 aufweist.

20. Verwendung einer kolloidalen stabilen Suspension strukturierter submikronischer Partikel, die mit einem oder mehreren Wirkprinzipien PA beaufschlagt sind, wobei diese Partikel in supramolekularen vereinigten (diskreten) Anordnungen vorliegen:
- auf Basis amphiphiler, linearer Polyaminosäuren (PAA) mit peptidischen Ketten mit mindestens 2 unterschiedlichen Typen wiederkehrender hydrophiler Aminosäuren AAI und hydrophober Aminosäuren AAO, wobei die Aminosäuren jeden Typs gleich oder verschieden voneinander sind;
- wobei sie sich dazu eignen, in kolloidaler Suspension im nicht gelösten Zustand mindestens ein PA anzulagern und dieses, insbesondere in vivo, verlängert und/oder verzögert erneut freizusetzen;
- und wobei sie in wässriger Phase bei einem pH-Wert von 4 bis 13 in Abwesenheit oberflächenaktiver Mittel stabil sind,
in denen:
die wiederkehrenden hydrophilen Aminosäuren AAI zumindest teilweise aus Asparagin zusammengesetzt
und die wiederkehrenden hydrophoben Aminosäuren AAO neutrale hydrophobe Aminosäuren AANO sind, die gleich oder verschieden voneinander sind,
zur Herstellung einer wässrigen Suspension oder eines pulverförmigen Feststoffs, die mit mindestens einem PA beaufschlagt und in den vorhergehenden Ansprüchen definiert sind.
